# EUROPEAN PATENT SPECIFICATION

(11) **EP 0 736 544 B1**
(45) Date of publication and mention of the grant of the patent: **24.10.2001**
(21) Application number: 95302271.2
(22) Date of filing: 05.04.1995
(51) Int. Cl.: C07K 16/18, A61K 47/48, A61K 7/16

(54) **Oral care compositions**
Mundpflegemittel
Compositions poursoins buccaux

(43) Date of publication of application: 09.10.1996
(73) Proprietor: UNILEVER PLC, London EC4P 4BQ (GB); UNILEVER N.V., 3013 AL Rotterdam (NL)
(72) Inventor: Cummins, Diane Unilever Port Sunlight Lab., Bebington, Wirral, Merseyside L63 3JW (GB); Pickup, Karen M. Unilever Port Sunlight Lab., Bebington, Wirral, Merseyside L63 3JW (GB); Tabak, Larry A. University of Rochester, Box 611, Rochester NY 14642 (US)
(74) Representative: van Gent, Jan Paulus

(56) References cited:
- DATABASE WPI Section Ch, Week 9132, Derwent Publications Ltd., London, GB; Class B04, AN 91-234074 & JP-A-3 151 892 (NIPPON SHOJI KK) 28 June 1991

## Description

### Field of Invention

The present invention relates to the delivery of therapeutic, eg. antimicrobial, or cosmetic, eg. anti-stain active agents to the tooth surface by the use of antibodies which specifically recognise protein structures (cryptitopes) on a pellicle, and to oral care compositions comprising such antibody systems as hereinafter described in more detail.

### Background of Invention

The acquired enamel pellicle is formed by the selective adsorption of salivary proteins onto a clean tooth surface (Bennick et al. (1979) Biochem. J. 183, 115-126; Bennick et al. (1983) Arch. Oral Biol. 28, 19-27; Rolla et al. (1983) Scand. J. Dent. Res. 91, 186-190; Al-Hashimi et al. (1989) Arch. Oral Biol. 34, 289-295).

It is thought that upon adsorption salivary proteins undergo a conformational change which results in the exposure of hidden structures, referred to as "cryptitopes" (Moreno et al. (1991) Biofouling 4, 3-24).

In-vitro studies suggest that several oral microorganisms, including *Actinomyces viscosus* (an organism that preferentially colonises the teeth and has been associated with gingivitis) have the ability to recognise such cryptitopes and in doing so bind efficiently to enamel resulting in microbial adhesion (Gibbons et al. (1990) Arch. Oral Biol. 35 (suppl.) 1075-1145). Plaque development frequently follows microbial adhesion which in the absence of good oral hygiene often results in a disease state, eg. caries, gingivitis.

Certain oral organisms, eg. *A. Viscosus, S.gordonii*, have been shown to bind avidly to proline-rich proteins (PRPs) on hydroxyapatite surfaces but not to PRPs in solution (Gibbons et al. (1988) Infect. Immun. 56, 439-445; Gibbons R.J. (1989) J. Dent. Res. 68, 750-760). Also, studies on FTIR spectra of PRPs in solution and in an adsorbed state have shown marked changes in FTIR spectra consistent with significant changes in beta-sheet and beta-turn contents of adsorbed proteins (Moreno et al. (1991) Biofouling 4, 3-24). The results of these studies suggest but do not confirm the cryptitope theory whereby proteins undergo a conformational change and new sites are exposed upon adsorption to a surface. Furthermore, it is not known whether changes in conformation or cryptitope expression on oral surfaces will provide new immunological targets or epitopes.

### Statement of Invention

The present invention relates to the use of monoclonal antibodies (murine) which exclusively recognise the surface bound form of a given pellicular constituent (cryptitope) on the acquired enamel pellicle. Such antibodies, which have been raised against a salivary pellicle, can be used to target "actives" to the pellicle.

The only other antibodies which have been shown to recognise protein on a salivary pellicle are anti-idiotype monoclonal antibodies that have been raised against presumptive adhesins of *S.sanguis.* Results here have shown the ability of the antibodies to mimic an *S.sanguis* adhesin with high specificity and to bind to an epitope that is a receptor for the *S.sanguis* adhesin on a salivary pellicle (Gong et al. (1993) J. Dent. Res. 72, Abs. 1766). However, these antibodies differ from those of the present invention in that they have not been raised against cryptitopes and it is not known if they do indeed recognise cryptitopes on a salivary pellicle.

Other research in this area includes the use of a novel genetically engineered binding protein which competes with a bacterial adhesin for a pellicle binding site. Here a pellicle is allowed to form and then sites are recognised by certain oral bacteria covered with the binding protein (WO-A-92/06192). This binding protein, however, has been engineered based on a particular amino acid sequence region of a salivary protein (PRP) in solution and not on a surface and would have thus not been conformational in structure.

Data that show the binding of human salivary components to oral strains of streptococcal bacteria (eg. Douglas et al., Archs. Oral Biol. 29, 751-757 (1984); Newman et al. (1993) Electrophoresis 14, 1322-1327; Newman et al. (1994) J. Dent. Res. 73, Abst. No. 396) suggest the presence of a salivary "pellicle" over bacterial surfaces within a biofilm.

Surprisingly, it has been found that the antibodies according to the present invention, in addition to recognising cryptitopes on model tooth surfaces, also recognise the same cryptitopes on bacterial pellicles. These antibodies are thus ideal tools to target actives to not only the acquired enamel pellicle but also to a salivary coat on a developing biofilm.

One of the major technical problems associated with the development of effective active systems for oral care benefits is obtaining substantive delivery of the agents to the desired site, eg. tooth where plaque formation occurs. Anti-plaque agents which are currently used are substantive where they bind to oral surfaces via (i) electrostatic (ii) hydrophobic interactions. However, binding which is reversible is non-specific i.e. agents will bind to all oral tissues.

The antibodies of the present invention can be used to target agents (eg. therapeutic, cosmetic) to a site which can serve as an anchor point within the mouth and thereby provide the necessary substantivity for maximum effect.

Although antibodies which have been raised against various oral bacteria eg. *S.mutans, S.sanguis* can be used to target anti-microbials to the bacterial surface in an attempt to break down plaque , the pellicle antibodies of the present invention differ from these antibodies in that in addition to their reactivity against a salivary coat on a developing biofilm where they can target actives to break-down plaque, they can also target actives to a clean tooth surface where they can prevent the build-up of plaque. Also, these antibodies can target a tooth whitening agent (or an agent which breaks down stain) to the site of extrinsic stain formation i.e. the acquired enamel pellicle.

Conjugates can be formed between the monoclonal antibodies of this invention and several active compounds including enzymes, peptides, antibodies, particulate systems, ligands, etc. These compounds can so be targeted to salivary pellicle on the tooth surface to control eg. plaque development, stain formation. Targeting and thus substantivity can lead to a reduced amount of "active" required. The conjugates can be formed chemically or through the tools of molecular biology.

### Detailed Description of the Invention

The purpose of the present invention is to use the monoclonal antibodies of the invention as targeting groups for the substantive delivery of oral care active agents, particularly antimicrobial and anti-stain actives, to the salivary pellicle on tooth and to the salivary coat on bacterial surfaces.

A first antibody or antibody fragment binds to a target site and one or more further antibodies or fragments collectively attach an agent to the target site. Conjugates are formed through either (i) chemical conjugation between pellicle antibodies (fragments) and an agent or (ii) recombinant DNA technology. Alternatively, a fusion can be made which consists of the antibody fragment binding region and the "active".

Finally, conjugates can be formed through non-covalent self assembly systems whereby (i) an anti-pellicle antibody or fragment thereof targets to the desired site and an other antibody (or fragment) system targets to the anti-pellicle antibody or (ii) hybrid antibodies or fragments target to pellicle and "pull down" an anti-microbial enzyme or other active compound (for example bispecific fragments with anti-pellicle and anti-glucose oxidase specifity).

The monoclonal antibodies can be raised against salivary pellicles that are formed from parotid, submandibular/sublingual and whole saliva.

An advantage of this invention is that pellicle antibodies which have been raised against a parotid saliva pellicle recognise their target in both parotid and whole saliva in-vitro formed pellicles. Another advantage is that such antibodies are substantive (i.e. withstand the flow of saliva), are not inhibited by soluble salivary components and remain reactive vs. their target in the presence of a developing biofilm. Another advantage is that such antibodies are reactive vs. an in-vivo formed pellicle. Such antibodies are ideal tools for targeting "actives" to the tooth surface. Such actives can be targeted to both a clean tooth surface i.e. the acquired enamel pellicle to prevent plaque or stain formation, or to a salivary coat on a developing bacterial biofilm to break-down plaque or stain formation.

Actives that can be used include:
(i) Antimicrobial: -
   (a) antimicrobial enzymes for examples oxidases including glucose oxidase, lactate oxidase, galactose oxidase, uric acid oxidase; peroxidases including horse radish peroxidase, myeloperoxidase, lactoperoxidase, chloroperoxidase; proteases including papain, pepsin, trypsin, ficin, bromelin; cell wall lytic enzymes including lysozyme; specific enzyme inhibitors such as those which inhibit neuraminidase activity or production; plaque matrix inhibitors including dextranases, mutanases.
   (b) antimicrobial peptides for example peptides including bacteriocins, histatins, defensins, cecropins.
   (c) Sequestering agents for example iron sequestering lactoferrin.
(ii) Cosmetic benefits-producing actives
   (a) Tooth whitening agents for example bleaching enzymes including glucose oxidase, myeloperoxidase; enzymes that break-down stain including proteases.
   (b) anti-malodour compounds including zinc and metal ion complexes.
(iii) Alkali generating systems including urease, arginine, deaminase, asparginase, etc..
(iv) Particulate systems including zinc oxide, latex, capsules, liposomes, emulsions.
(v) Redox buffers

Preferred systems include targeted enzymes (enzyme substrates in gels): targeted enzymes in rinse (substrates in gels); targeted enzymes and substrates in rinse; targeted enzymes and substrates in pastes; targeted enzyme and substrates in lozenges.

The recommended concentration of the targeted active-agent/antibody in a product will be from 0.01 *µ*g/g to 50 mg/ml.

The oral compositions can be formulated in any suitable application form, such as emulsions, gels, mouthwashes, toothpowders and toothpastes. They may be formulated into a single formulation or they may be formulated for multi compartment containers into different formulations.

The oral care compositions may, furthermore, comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients.

Thus, they may comprise particulate abrasive materials including agglomerated particulate abrasive materials such as silicas, aluminas, calcium carbonates, dicalciumphosphates, calcium pyrophosphates, hydroxyapatites, trimetaphosphates, insoluble hexametaphosphates and so on, usually in amounts between 5 and 60 % by weight.

Furthermore, the dentifrice formulations may comprise humectants such as glycerol, sorbitol, propyleneglycol, polyethyleneglycol, xylitol, lactitol and so on.

Binders and thickeners such as sodium carboxymethyl-cellulose, xanthan gum, gum arabic etc. may also be included, as well as synthetic polymers such as polyacrylates and carboxyvinyl polymers such as Carbopol® .

Flavours such as peppermint and spearmint oils may also be included, as well as preservatives, opacifying agents, colouring agents, pH-adjusting agents, sweetening agents and so on.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of additional anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Desensitising agents such as potassium citrate, potassium chloride, potassium tartrate, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise further functional biomolecules such as bacteriocins, antibodies, enzymes and so on.

Other optional ingredients that may be included are e.g. bleaching agents, e.g. those described in EP-A-0 545,594, organic peroxyacids, effervescing systems such as sodium bicarbonate/citric acid systems, colour change systems, and so on.

When formulated as a mouthwash, the oral care composition usually comprises a water/alcohol solution, flavour, humectant, sweetener and colorant.

The present invention will further be illustrated by way of Example.

### Example 1

### Generation of Cryptitope - Specific Monoclonal Antibodies

### Method

### 1) Immunisation

Immunogen - One ml of freshly collected parotid saliva was added to 35 mg of pre-washed (washed with phosphate buffered saline: PBS pH 7.4) hydroxyapatitie (HA) for two hours at 4°C with end over end mixing. Parotid saliva coated HA was then washed (PBS) and centrifuged X2 (2000 g). The resultant pellet was mixed with either Freund's adjuvant or Titre adjuvant to increase the immune response prior to administration of the vaccine to mice (day 1). Mice were re-injected with the above immunogen on day 8. The immune response of the mice was boosted on days 15, 22, 30 by injection of parotid saliva coated HA. A fusion was performed on day 35.

Cell fusion - The spleen from an immunised mouse was removed and placed in 30 ml of serum free RPMI 1640 media to form a single cell suspension. The spleen cells were then fused with a myeloma cell line using polyethylene glycol. Cells were then dispersed into HAT (Hypoxanthine, Aminopterin, Thymidine)media (a media that is used to promote conditions which allows the growth of hybrid cells only) and aliquoted into 96 well tissue plates.

### 2) Screening Assay - Detection of antibodies with specificity for surface bound determinants on parotid saliva coated HA.

PBS washed HA was added to freshly collected parotid saliva at a concentration of 35 mg HA/ml of parotid saliva. The sample was then mixed overnight end over end at 4°C. Following this, the sample was washed (PBS) and centrifuged (2000 g) X2 prior to the addition of 5 % Bovine Serum Albumin (BSA) in PBS (PBSA). Saliva coated HA was then mixed end over end for one hour at 4°C. The sample was then washed as above before PBSA was added to the HA pellet to a concentration of 5 mg HA/100 *µ*l PBSA. Following this, 100 *µ*l aliquots of the HA sample were added to Eppendorf® tubes. PBSA (150 *µ*l) or fresh parotid saliva (150 *µ*l) was then added to HA prior to the addition of antibody (100 *µ*g/ml).

Samples were incubated at room temperature for a period of 30 minutes and vortexed twice (time 0.15 minutes) during this time. Samples were washed and centrifuged X3 with 500 *µ*l of 0.05 % Tween® 20 in PBS (PBST). A secondary antibody labelled with Horse Radish Peroxidase (HRP)was then added (250 *µ*l, 1/1000 dilution) for 30 minutes at room temperature (sample was vortexed at time 0 and 15 minutes as above). Following this, the samples were again washed and centrifuged X3 with 500 *µ*l PBST prior to the addition of 250 *µ*l of the substrate TMB (3,3',5,5' Tetramethylbenzidine) (4 minutes, RT). The reaction was stopped via the addition of 2M H₂SO₄ (250 *µ*l). Finally, 400 *µ*l of each reaction mixture was filtered and the filtrate transferred to an ELISA plate where readings were taken on a spectrophotometer at an absorbance of 450ₙₘ

### Results

### Generation of Cryptitope - Specific Monoclonal Antibodies

Numerous cell lines were screened for antibody production in the above modified ELISA screen. Cell lines that were positive were cloned and then "grown up" to mg quantities in hollow cell fibre reactors.

Table I illustrates ELISA results from cloned and grown up monoclonal antibodies where antibodies have reacted against saliva coated HA in the presence of both buffer and saliva. The fact that saliva has not inhibited or reduced the activity of the antibodies suggests that the antibodies are preferentially recognising adsorbed salivary protein i.e. cryptitopes. These antibodies are thus the first antibodies to recognise cryptitopes.

**Table 1**

| Monoclonal Antibodies | Binding to saliva coated HA in *buffer* | Binding to saliva coated HA in *saliva* |
|---|---|---|
| 1A1 | 0.43 | 0.44 |
| 1C6 | 0.35 | 0.36 |
| 1D3 | 0.42 | 0.40 |
| 3D5 | 0.40 | 0.40 |
| Negative Control = 0.1 (NB The monoclonal antibodies were all of the IgG isotype class). | | |

### Example 2

### Binding of Monoclonal Antibodies to Pellicle and Developing Plaque in-vitro

### Method

### 1) Immunospecificity of monoclonal antibodies vs. both parotid and whole saliva pellicles

Immunospecificity of the antibodies v.s. both parotid and whole pellicles was determined using confocal laser scanning microscopy (CLSM). This i method was selected because (i) it has a different detection step to that of the above ELISA and so was another means of confirming specificity v.s. parotid saliva pellicles in addition to testing reactivity of antibodies v.s. whole saliva pellicles and (ii) allowed direct in-situ viewing of the reaction on saliva HA discs. This was particularly advantageous in time-course studies (see 3 below).

Experiment - The pellicle was formed by coating HA discs at 4°C in 2.5 ml of saliva overnight with parotid saliva or for 2 hours with whole saliva (to prevent protein degradation) on a rocking platform STR6 (Stuart) at maximum speed.

The discs were then washed (PBS) prior to the addition of a blocking agent (one hour at RT) to reduce non-specific reactions. The blocking agent used throughout all these studies was 5 % BSA as this proved to effectively block and is found naturally in saliva. Following the blocking step the discs were again washed before the addition of the pellicle monoclonal antibody (30 minutes).

After a further wash, a secondary fluorescently labelled (FITC) antibody (Vector) was added (1/20 dilution, 30 minutes). Finally the discs were washed and viewed under the CLSM.

### 2) Reactivity of monoclonal antibodies v.s. protein constituents of parotid and whole saliva in a Western Blot.

The specificity of the monoclonal antibodies was also examined by a Western Blot of salivary protein.

Experiment - Aliquots of parotid and whole saliva were subjected to SDS PAGE (Laemmli UK (1970) Nature 227, 680) for 1.8 hours at 80 volts before separated proteins were transferred onto nitrocellulose membranes (pore size 0.45 *µ*m, Sigma) by a semi-dry transfer unit (Biorad) for 5 hours (5 volts). Membranes were stained with Ponceau S. (Sigma) to confirm a successful transfer. Membranes were then blocked with 5 % BSA (4°C) for 2 hours before anti-pellicle antibodies were added (RT) for a further 2 hours. Reactivity was detected by the addition of a secondary antibody conjugated to HRP (RT; 2 hours) followed by the substrate (4-Chloro-1-Naphthol, Sigma) which allowed the visualisation of antibody-protein bands.

(NB Antibodies were tested v.s. reduced and non-reduced salivary proteins on acrylamide gels of 5-15 %. Low molecular weight SDS PAGE standards (Biorad) were used in all Western Blot procedures to allow size approximation of protein-antibody bands).

### 3) Time-Course Studies

An in-vitro model was used to simulate the oral cavity to study (i) substantivity of the antibodies and (ii) anti-pellicle binding to a more mature pellicle in the absence/presence of bacteria. Pellicles were formed on HA discs as described earlier. The models were fed from 50 ml reservoir flasks which were filled with saliva and pumped through at 6 ml/hour. All model experiments were carried out at 37°C. Prepared HA discs were inserted into modified 1ml pipette tips before being transferred to the model. After the required incubation time, the discs were removed and labelled with antibody. FITC binding was quantified by CLSM.

### Experiment (i) Effects of saliva flow on anti-pellicle antibody binding (Substantivity Determination)

Pellicles were formed using parotid saliva. Anti-pellicle antibodies were added to the HA discs for 30 minutes prior to incubation in the model. The discs were placed in the model under a constant flow of saliva and removed at hourly intervals for up to 4 hours. Fluorescently labelled antibody was added and FITC binding quantified by CLSM as before.

### Experiment (ii) Anti-pellicle antibody binding to a more mature pellicle

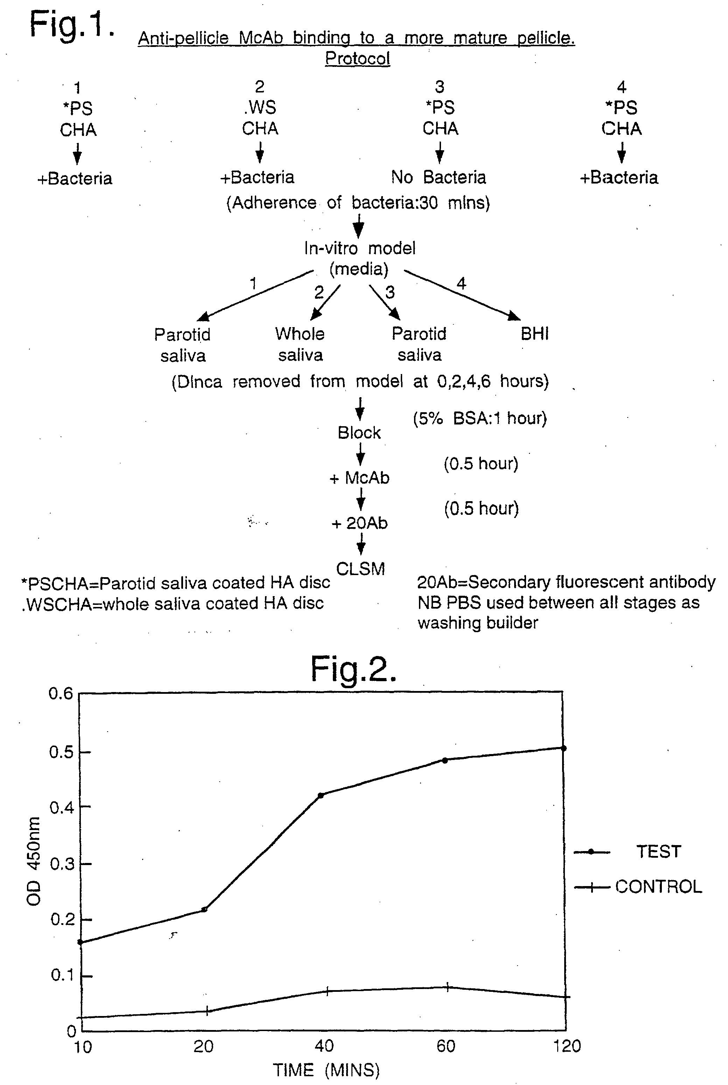

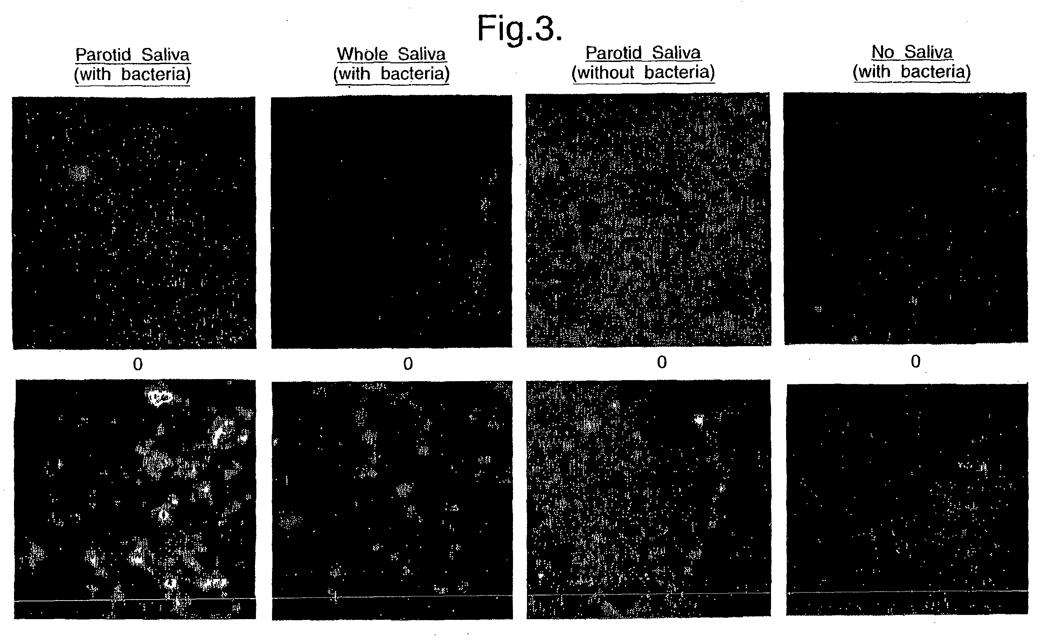

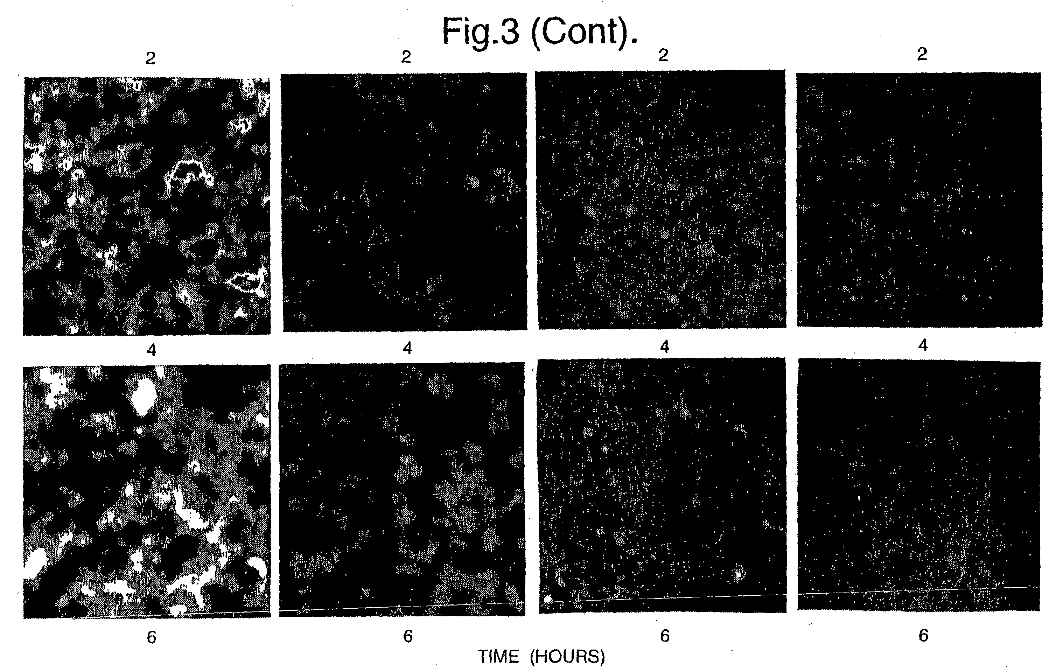

Pellicles were formed using whole and parotid saliva. The effects on binding antibody against saliva coated HA discs incubated in the model for up to 6 hours was assessed. Models were incubated with (see * for preparation of bacteria) and without bacteria. Also models were ran where Brain Heart Infusion media replaced saliva. The discs were removed from the model (time 0, 2, 4 and 6 hours) prior to the addition of anti-pellicle antibody followed by the fluorescently labelled antibody. FITC binding was quantified by CLSM. The full protocol for this experiment is outlined in Figure 1.

Preparation of micro-organisms - The organisms used to produce a 3 bacterial biofilm were *Actinomyces viscosus, Streptococcus mutans* SM211 and *Streptococcus oralis*: a streptomycin resistant strain. The organisms were grown on Brain Heart Infusion (BHI) supplemented agar (BHI:Oxoid 47 g/l, Yeast Extract:Oxoid 5 g/l, Cysteine HCL:Sigma 0.1 g/l, Haemin:Sigma 500 mg/l, Vitamin K:Sigma 5 mg/l and sterile horse blood:Oxoid 50 ml/l) before colonies were taken aseptically to inoculate sterile BHI broths. After an overnight incubation the broths were centrifuged (2000 g:10 minutes) and washed 3 times in PBS before bacterial suspensions were corrected to OD600ₙₘ of 1.0.

### Results

### Binding of Monoclonal Antibodies to Pellicle and Developing Plaque in-vitro

### 1) Immunospecificity of antibodies versus parotid and whole saliva pellicles

All the antibodies were found to be immunospecific versus parotid saliva pellicles and to a lesser extent versus whole saliva pellicles. The reduction in activity is likely due to either: (i) the epitope the antibody is targeted to not being as abundant in whole saliva pellicles as in parotid saliva pellicles or (ii) components in whole saliva may be masking the epitope.

### The results are shown in Table 2:

### Specificity of pellicle McAbs versus parotid and whole saliva pellicles

| Antibody Binding Pixel Intensity* | | |
|---|---|---|
| Pellicle McAb | Partorid saliva pellicle | whole saliva pellicle |
| 1D3 | 62.67 (+/- 5.58) | 20.67 (+/-3.16) |
| 1C6 | 54.08 (+/- 8.23) | 13.45 (+/-2.61) |
| 1A1 | 61.49 (+/- 7.27) | 13.90 (+/-3.02) |
| 3D5 | 56.32 (+/-14.94) | 22.60 (+/-2.40) |
| Neg. control (FITC Ab only) | 10.12 (+/- 1.80) | 9.98 (+/-0.50) |

| | | |
|---|---|---|
| * measure of fluorescence = pixel intensity | | |

(Other IgG antibodies tested = comparable to negative control )

### 2) Reactivity of antibodies v.s. immobilised proteins: Western Blot

All the antibodies reacted against protein bands of 50, 100 and 200 KDa in both parotid and whole saliva. It is quite probable that the salivary proteins have behaved in a similar manner on nitrocellulose membranes as they do on hydroxyapatite where cryptitopes have been exposed.
The results are shown in **Table 3**:

**Table 3**

| Western Blot of Antibodies v.s. Salivary Proteins | |
|---|---|
| McAb | McAb Reactivity v.s. Protein Bands (KDa) |
| 3D5 | 50, 100, 200 |
| 1A1 | 50, 100, 200 |
| 1C6 | 50, 100, 200 |
| 1D3 | 50, 100, 200 |
| Neg. Controls | Negative |

### 3) Time-Course Studies

### (i) Substantivity Determination

No significant reduction in activity of the antibodies was observed when saliva was allowed to flow for up to 4 hours over the pellicle coated HA discs. This result indicates substantivity of the antibodies.
The results are shown in **Table 4:**

### Substantivity of pellicle McAbs (a typical result)

| Time (Hrs) | Antibody Binding Pixel Intensity | |
|---|---|---|
| | Pellicle McAb | Negative Control |
| | (no pellicle McAb) | |
| 0 | 43.6 (+/- 9.8) | 13.6 (+/- 0.9) |
| 1 | 37.9 (+/- 2.2) | 12.2 (+/- 0.9) |
| 2 | 35.4 (+/- 6.4) | 9.0 (+/- 1.1) |
| 3 | 38.2 (+/- 8.9) | 10.2 (+/- 0.7) |
| 4 | 31.1 (+/- 7.3) | 10.5 (+/- 1.3) |

### (ii) Anti-pellicle binding to a more mature pellicle in the absence/presence of bacteria.

An increase in fluorescence i.e. anti-pellicle binding over time (up to 6 hours) was observed when antibodies were tested against a developing biofilm on both parotid and whole saliva pellicles, as shown in the attached i photopgraphs, nos. 1 and 2. The increase in antibody binding was not solely due to (i) increase in site caused by the development of a more extensive pellicle as an increase in fluorescence was not observed in a sterile control i.e. parotid saliva, no bacteria (photograph no. 3) or (ii) metabolising organisms as no increase in fluorescence was observed when BHI replaced saliva in the model (photograph no. 4). It thus appears that this response is due to a combination of salivary protein and bacteria where the antibodies have reacted with adsorbed salivary proteins (i.e. potential cryptitopes) on a developing biofilm. This result was very surprising as it was not expected that the antibodies would recognise cryptitopes on bacterial surfaces to cryptitopes on a HA surface.
(NB It must be noted that negative controls resulted in a totally black image on the CLSM).

All 4 antibodies have proved to be immunospecific vs. parotid saliva pellicles and to a lesser extent vs. whole saliva pellicles. The similarity in binding behaviour and reactivity on Western Blots of these antibodies suggests that they recognise the same antigenic site. These antibodies have much potential as they have proved to be substantive and to react increasingly so with the salivary coat on a developing biofilm. This latter result was very exciting where a developing biofilm enhances antibody targeting. The implications of this are great with respect to the delivery of a functionality i.e. antibodies will be able to target and deliver "actives" to (i) proteins on the acquired enamel pellicle to prevent the build up of e.g. plaque and stain and to (ii) proteins on a salivary coat on a microbial biofilm to break down eg. plaque and stain.

### Example 3

### Binding of Monoclonal Antibodies to an In-situ formed Pellicle Method

The reactivity of antibodies vs. protein structures on an in-situ formed pellicle was investigated. A device, similar to a palate that held 8-10 enamel slabs was used to form the in-situ pellicles. The enamel slabs were held in a soft resin on the device to allow easy removal of the slabs (without disturbing the pellicle) following the required period of pellicle formation.

Experiment - The device was placed in the mouth for 5 hours to allow the development of an enamel pellicle. During this time period microbial colonisation (and a salivary coat on bacterial surfaces) would be expected to commence. Following this period of incubation the device was removed from the mouth. The enamal slabs were carefully removed from the soft resin prior to washing (PBS) and the addition of the monoclonal antibody (30 minutes). After a further wash, the fluorescently labelled (FITC) antibody was added (30 minutes) before enamel slabs were washed and viewed under the CLSM.

### Results

The results are shown in **Table 5:**

### Reactivity of pellicle McAbs versus in-situ formed pellicles

| Pellicle McAb | Antibody Binding Pixel Intensity |
|---|---|
| 1D3 | 105.24 (+/- 10.14) |
| 1C6 | 98.46 (+/-8.10) |
| 1A1 | 110.08 (+/-15.10) |
| 3D5 | 103.29 (+/-11.29) |
| Neg. control | 14.25 (+/-2.11) |

All 4 antibodies were reactive vs. an in-situ formed pellicle (table 5 ). This result was very promising where during the time period of pellicle formation, the antibodies have likely reacted against cryptitopes on the aquired enamel pellicle in addition to adsorbed protein (potential cryptitopes) on bacterial surfaces. The results here indicate the usefulness of anti-pellicle antibodies as a tool to deliver 'actives' to a surface.

### Example 4

### Targeting of Oxidative Enzymes by Monoclonal Antibodies In-vitro Method

The ability of the antibodies to target an oxidative enzyme (glucose oxidase: GOX) to an in-vitro formed pellicle was investigated. The delivery of glucose oxidase was determined by measuring hydrogen peroxide produced by GOX in the presence of 5% glucose. A parotid saliva pellicle was formed by shaking parotid saliva over hydroxyapatite discs at 4°C overnight. The discs were washed (PBS), blocked with 5% BSA and washed again prior to the addition of monoclonal antibody (30 minutes). Following this, a GOX labelled secondary antibody (Organon Teknika Corporation) was added (30 minutes). The discs were then washed prior to the addition of TMB (Sigma) substrate* (2ml/disc) which was shaken over the discs at RT on a Stuart STR6 rocking platform (maximum speed). Finally, the total volume of solution was aliquoted into an ELISA plate and absorbances read on a spectrophotometer at 450ₙₘ.

(*Substrate solution: 5% glucose, 10 ml phosphate citrate buffer (0.075M citric acid; 0.316M sodium hydrogen phosphate), 100 *µ*l TMB/Dimethyl Sulphoxide : 0.1g TMB, 10ml dimethyl sulphoxide, 10 *µ*l HRP (0.5mg/ml) NB: no hydrogen peroxide).

### Results

### Targeting of Oxidative Enzymes by Monoclonal Antibodies In-vitro

The results are shown in the attached Figure 2.
The ability of the anti-pellicle antibodies to target GOX to a surface was confirmed ( table 6 ) . The ability of the anti-pellicle antibodies to deliver was confirmed by the lack of GOX delivery by the control where there was no anti-pellicle antibody (ie GOX labelled antibody and substrate only). This result was extremely promising where the anti-pellicle antibody has tethered the GOX labelled antibody and in doing so delivered the GOX to a surface. GOX is a useful 'active' where hydrogen peroxide produced from GOX can be used as a (i) bleach to whiten teeth or (ii) an antimicrobial (plus a substrate for a further antimicrobial) to control the development of plaque.

### Example 5

### Delivery of Antibacterial Activity by Monoclonal Antibodies In-situ Method

In this experiment anti-pellicle antibodies were studied for their ability to deliver GOX and HRP labelled antibodies (Organon Teknika Corporation) to a surface, in this case an in-situ formed pellicle. GOX, in the presence of glucose produces hydrogen peroxide which is used by HRP to oxidise thiocyanate or potassium iodide (KI) to produce antibacterial ions. The substantivity of the antimicrobials is improved by using anti-pellicle antibodies to tether GOX and HRP labelled antibodies to a surface.

Experiment - A pellicle was formed in-situ over a time period (5 hours) which allowed microbial colonisation. The pellicle was formed using the device described earlier (see example 3). After 5 hours incubation the device was removed from the mouth and the enamel slabs gently eased from the soft resin. The slabs were then washed (PBS) and blocked (5% BSA) prior to the addition of the anti-pellicle antibody (30 minutes) followed by the GOX (1/500) and HRP (1/500) labelled antibodies (30 minutes). Enamel slabs were then exposed to substrate (5% glucose, 1mM KI) for I hour at 37°C (CO₂ incubator). Finally samples were plated out and colony forming units/ml (CFU's/ml) calculated.

### Results

### Delivery of Antibacterial Activity by Monoclonal Antibodies In-situ

The results are shown in **table 7:**

| Delivery of anti-microbials by pellicle McAbs | |
|---|---|
| Treatment ( vs. bacteria on enamel slabs) | Viable Count Log cfu's /ml |
| Test | 1.30 (+/- 0.02) |
| Pellicle McAb + GOx /HRP Abs +substrates | |
| | |
| Control 1 | 3.30 (+/-0.30) |
| GOx / HRP Abs + substrates ( no pellicle Ab) | |
| | |
| Control 2 | 3.37 (+/- 0.90) |
| Pellicle McAb + substrates | |
| | |
| Control 3 | 3.24 (+/- 0.15) |
| Substrates only | |

The test ie anti-pellicle antibody, GOX and HRP antibodies plus substrate resulted in the targeted delivery of antimicrobials where a 1 to 2 log reduction of bacterial CFU's/ml was observed compared to negative controls (table 7 ). The delivery of these antimicrobials has likely been enhanced by the ability of the antibodies to recognise two sites ie the acquired enamel pellicle and the salivary coat on a developing biofilm. Anti-pellicle antibodies, therefore, appeared to be excellent tools to target and deliver therapeutic or cosmetic actives to the tooth surface.

## Claims

1. Monoclonal antibodies, raised against salivary pellicle, capable of recognising cryptitopes.

2. Monoclonal antibodies according to claim 1, conjugated with a therapeutic or cosmetic active agent.

3. Monoclonal antibodies according to claim 2, conjugated with an oxidatve enzyme.

4. Oral care compositions comprising an antibody according to claims 1-3.

5. Use of monoclonal antibodies, raised against salivary pellicle, capable of recognising cryptitopes, in the preparation of oral care compositions as defined in claim 4 for targeting anti-microbial or anti-stain active ingredients to the enamel pellicle or to the salivary coat on a developing biofilm on the teeth.

## Patentansprüche

1. Monoklonale Antikörper gegen Speichelbelag, die Cryptitope erkennen können.

2. Monoklonale Antikörper nach Anspruch 1, konjugiert mit einem therapeutisch oder kosmetisch aktiven Mittel.

3. Monoklonale Antikörper nach Anspruch 2, konjugiert mit einem oxidativen Enzym.

4. Mundpflegezusammensetzungen enthaltend einen Antikörper nach einem der Ansprüche 1 bis 3.

5. Verwendung von monoklonalen Antikörpern gegen Speichelbelag, die Cryptitope erkennen können, zur Herstellung von Mundpflegezusammensetzungen, wie in Anspruch 4 definiert, um antimikrobielle oder Antifleck-aktive Inhaltsstoffe dem Schmelzbelag oder dem Speichelüberzug auf einem sich entwickelnden Biofilm auf den Zähnen gezielt zuzuführen.

## Revendications

1. Anticorps monoclonaux, élevés contre la pellicule salivaire, capables de reconnaître des cryptitopes.

2. Anticorps monoclonaux selon la revendication 1, conjugués avec un agent actif thérapeutique ou cosmétique.

3. Anticorps monoclonaux selon la revendication 2, conjugués avec une enzyme oxydative.

4. Compositions pour soins buccaux comprenant un anticorps selon les revendications 1 - 3.

5. Utilisation des anticorps monoclonaux, élevés contre la pellicule salivaire, capables de reconnaître des cryptitopes, dans la préparation des compositions pour soins buccaux telles que définies dans la revendication 4, pour cibler des agents antimicrobiens ou des ingrédients actifs anti-taches sur la pellicule d'émail ou le revêtement salivaire sur un biofilm de développement sur les dents.
